# EUROPEAN PATENT APPLICATION

(11) **EP 4 563 974 A1**
(43) Date of publication of application: **04.06.2025**
(21) Application number: 24214794.0
(22) Date of filing: 22.11.2024
(51) Int. Cl.: G01N 21/27, G01N 21/78, G01N 33/92, G01N 35/00, G01N 1/28, G01N 21/25

(54) **EVALUATION APPARATUS, SYSTEM, AND EVALUATION METHOD**

(30) Priority: 30.11.2023 JP 2023202798
(71) Applicant: Jeol Ltd., Akishima-shi, Tokyo 196-8558 (JP)
(72) Inventor: KASHIWAGI, Yasutoshi, Tokyo, 196-8558 (JP); ABE, Kentaro, Tokyo, 196-8558 (JP); TANIMIZU, Akinori, Tokyo, 196-8558 (JP)
(74) Representative: Boult Wade Tennant LLP

(57) **Abstract**

An evaluation apparatus (40) includes: an acquiring unit (402) configured to acquire information on a change amount of an absorbance per unit time, the change amount being obtained by a measurement method including causing a peroxidase to act on hydrogen peroxide, which is produced by solubilizing an LDL in a specimen with a surfactant that acts on the LDL, and subjecting the resultant to an oxidation reaction with a cholesterol oxidase, to introduce the hydrogen peroxide to a color reaction, followed by measurement of a change of the absorbance with time; and an evaluating unit (404) configured to evaluate an amount of an oxidized LDL in the specimen based on the change amount.

## Description

### BACKGROUND OF THE INVENTION

### Field of the Invention

The present invention relates to an evaluation apparatus, a system, and an evaluation method.

### Description of Related Art

In the field of a clinical test, a biochemical autoanalyzer that measures the amount of a specific component in a specimen speedily and automatically has been widely disseminated. An extremely large amount of impurities are present in the specimen, and hence, in order to detect a target compound from the specimen, an antibody that selectively binds to the target compound or an enzyme that selectively reacts with the target compound is used. A detection reagent for detecting the target compound in which such antibody and enzyme are combined has been developed by a reagent manufacturer. To perform high-sensitivity and rapid measurement, such reagent has been devised, for example, as follows: a multistage reaction is performed in one pot. In, for example, Japanese Patent Application Publication No. Hei 10-38888 or Japanese Patent Application Publication No. Hei 9-313200, there is a disclosure of a method of determining the amount of low-density lipoprotein cholesterol (LDL-C) with such reagent.

A lipid taken in by a small intestine or a lipid biosynthesized in a liver (a fatty acid or cholesterol) is brought into the state of a triacylglyceride or a cholesteryl ester, and is transported as a spherical molecule called a lipoprotein, which is obtained as follows, to any other tissue through blood: the periphery of the triacylglyceride or the cholesteryl ester is covered with a phospholipid, cholesterol, and an apoprotein so that the triacylglyceride or the cholesteryl ester may be stabilized (see "Structure and Function of Plasma Lipoproteins," Journal of Japan Oil Chemists' Society, 40(10) 858-868 (1991)).

The lipoproteins are classified into a chylomicron (CM), a very low-density lipoprotein (VLDL), a low-density lipoprotein (LDL), and a high-density lipoprotein (HDL), which are arranged in order of increasing density, in accordance with differences in size, density, and apoprotein composition. The amount of cholesterol included in such lipoprotein has been widely measured as an indicator of each of various lipid-related diseases in the field of a clinical test.

The value of the LDL-C out of those lipoproteins is a risk factor of arteriosclerosis, and hence a therapy for reducing the amount of the LDL-C with a statin-based drug or the like has been widely disseminated (see "Oxidatively Modified Lipoprotein involved in Onset and Progression of Arteriosclerosis," Journal of The Showa University Society, 81(5) 370-379 (2021)). However, other risk factors have been pointed out because of, for example, the following reasons: a correlation between the LDL-C value and the onset of a disease is not necessarily high; and even when the LDL-C value is reduced, the risk of the onset cannot be completely eliminated.

Oxygen, which is required for the maintenance of a life, is changed into active oxygen by an external stimulus to serve as a cell transmitter or an immune function. Meanwhile, excessive production of oxygen damages a cell to serve as a factor causing various diseases, such as a cancer, a cardiovascular disease, and a lifestyle-related disease. Lipoproteins receive oxidative stresses to be responsible for those diseases. Of the lipoproteins, an oxidatively modified LDL (hereinafter also referred to as "oxidized LDL") has been considered to be one factor of the onset of arteriosclerosis because the oxidized LDL is taken in a cell such as a macrophage to cause the foaming of the cell. The amount of the oxidized LDL serves as an important indicator of a disease resulting from a change in function of a vascular endothelial cell, such as arteriosclerosis or a coronary syndrome.

A method including subjecting an oxidized LDL to colorimetry by a sandwich enzyme-linked immunosorbent assay (ELISA) method has been known as a method of measuring the amount of the oxidized LDL. In, for example, Japanese Patent No. 3561218, there is a disclosure of a diagnostic kit that detects an oxidatively modified lipoprotein containing ApoB 100 in blood by the sandwich ELISA method.

However, in the above-mentioned sandwich ELISA method, complicated treatment is required, and hence rapid measurement cannot be performed.

### SUMMARY OF THE INVENTION

According to a first aspect of the present disclosure, there is provided an evaluation apparatus including:
an acquiring unit configured to acquire information on a change amount of an absorbance per unit time, the change amount being obtained by a measurement method including causing a peroxidase to act on hydrogen peroxide, which is produced by solubilizing an LDL in a specimen with a surfactant that acts on the LDL, and subjecting the resultant to an oxidation reaction with a cholesterol oxidase, to introduce the hydrogen peroxide to a color reaction, followed by measurement of a change of the absorbance with time; and
an evaluating unit configured to evaluate an amount of an oxidized LDL in the specimen based on the change amount.

According to a second aspect of the present disclosure, there is provided a system including:
a measuring unit configured to cause a peroxidase to act on hydrogen peroxide, which is produced by solubilizing an LDL in a specimen with a surfactant that acts on the LDL, and subjecting the resultant to an oxidation reaction with a cholesterol oxidase, to introduce the hydrogen peroxide to a color reaction, followed by measurement of a change of an absorbance with time to output data on the change of the absorbance with time;
a differential operation unit configured to determine a change amount of the absorbance per unit time from the data; and
an evaluating unit configured to evaluate an amount of an oxidized LDL in the specimen based on the change amount.

According to a third aspect of the present disclosure, there is provided an evaluation method including:
acquiring information on a change amount of an absorbance per unit time, the change amount being obtained by a measurement method including causing a peroxidase to act on hydrogen peroxide, which is produced by solubilizing an LDL in a specimen with a surfactant that acts on the LDL, and subjecting the resultant to an oxidation reaction with a cholesterol oxidase, to introduce the hydrogen peroxide to a color reaction, followed by measurement of a change of the absorbance with time; and
evaluating an amount of an oxidized LDL in the specimen based on the change amount.

According to a fourth aspect of the present disclosure, there is provided an evaluation method including:
causing a specimen containing a first substance and a second substance, and a reagent to react with each other, followed by measurement of a change amount of a physical quantity of reaction products per unit time, the reaction products including a first reaction product produced by a first reaction between the first substance and the reagent, and a second reaction product produced by a second reaction between the second substance and the reagent; and
evaluating a physical quantity of the second substance based on the change amount.

### BRIEF DESCRIPTION OF THE DRAWINGS

FIG. 1 is a diagram for describing a first step of a method of measuring the amount of LDL cholesterol.
FIG. 2 is a diagram for describing a second step of the method of measuring the amount of LDL cholesterol.
FIG. 3 is a diagram for describing a method of evaluating the amount of an oxidized LDL.
FIG. 4 is a graph illustrating results of differential measurement of an oxidized LDL.
FIG. 5 is a graph illustrating results of measurement of absorbance of an oxidation-treated specimen before its dialysis.
FIG. 6 is a graph illustrating results of measurement of absorbance of an oxidation-treated specimen after its dialysis.
FIG. 7 is a table illustrating conditions for production of oxidation-treated specimens A, B, C, D and E.
FIG. 8 is a table illustrating incubation times and reaction delay indicators of the oxidation-treated specimens.
FIG. 9 is a histogram illustrating the reaction delay indicator for each incubation time.
FIG. 10 is a table illustrating the incubation times, reaction delay indicators, LDL cholesterol concentrations, and oxidized LDL ratios of the oxidation-treated specimens.
FIG. 11 is a histogram illustrating the reaction delay indicator and the LDL cholesterol concentration for each incubation time.
FIG. 12 is a histogram illustrating the oxidized LDL ratio for each incubation time.
FIG. 13 is a table illustrating incubation times and reaction delay indicators of oxidation-treated specimens.
FIG. 14 is a histogram illustrating the reaction delay indicator for each incubation time.
FIG. 15 is a diagram illustrating a configuration of an evaluation system according to an embodiment of the invention.
FIG. 16 schematically illustrates an example of a configuration of an autoanalyzer.
FIG. 17 is a diagram illustrating an example of a configuration of an evaluation apparatus.
FIG. 18 is a flowchart illustrating an example of processing of the evaluation apparatus.

### DESCRIPTION OF THE INVENTION

According to an embodiment of the present disclosure, there is provided an evaluation apparatus including:
an acquiring unit configured to acquire information on a change amount of an absorbance per unit time, the change amount being obtained by a measurement method including causing a peroxidase to act on hydrogen peroxide, which is produced by solubilizing an LDL in a specimen with a surfactant that acts on the LDL, and subjecting the resultant to an oxidation reaction with a cholesterol oxidase, to introduce the hydrogen peroxide to a color reaction, followed by measurement of a change of the absorbance with time; and
an evaluating unit configured to evaluate an amount of an oxidized LDL in the specimen based on the change amount.

In such evaluation apparatus, the amount of the oxidized LDL in the specimen can be evaluated based on the change amount of the absorbance per unit time measurable with an autoanalyzer. Accordingly, in such evaluation apparatus, the amount of the oxidized LDL can be rapidly evaluated.

According to an embodiment of the present disclosure, there is provided a system including:
a measuring unit configured to cause a peroxidase to act on hydrogen peroxide, which is produced by solubilizing an LDL in a specimen with a surfactant that acts on the LDL, and subjecting the resultant to an oxidation reaction with a cholesterol oxidase, to introduce the hydrogen peroxide to a color reaction, followed by measurement of a change of an absorbance with time to output data on the change of the absorbance with time;
a differential operation unit configured to determine a change amount of the absorbance per unit time from the data; and
an evaluating unit configured to evaluate an amount of an oxidized LDL in the specimen based on the change amount.

In such system, the amount of the oxidized LDL in the specimen can be evaluated based on the change amount of the absorbance per unit time measurable with the autoanalyzer. Accordingly, in such system, the amount of the oxidized LDL can be rapidly evaluated.

According to an embodiment of the present disclosure, there is provided an evaluation method including:
acquiring information on a change amount of an absorbance per unit time, the change amount being obtained by a measurement method including causing a peroxidase to act on hydrogen peroxide, which is produced by solubilizing an LDL in a specimen with a surfactant that acts on the LDL, and subjecting the resultant to an oxidation reaction with a cholesterol oxidase, to introduce the hydrogen peroxide to a color reaction, followed by measurement of a change of the absorbance with time; and
evaluating an amount of an oxidized LDL in the specimen based on the change amount.

In such evaluation method, the amount of the oxidized LDL in the specimen can be evaluated based on the change amount of the absorbance per unit time measurable with the autoanalyzer. Accordingly, in such evaluation method, the amount of the oxidized LDL can be rapidly evaluated.

According to an embodiment of the present disclosure, there is provided an evaluation method including:
causing a specimen containing a first substance and a second substance, and a reagent to react with each other, followed by measurement of a change amount of a physical quantity of reaction products per unit time, the reaction products including a first reaction product produced by a first reaction between the first substance and the reagent, and a second reaction product produced by a second reaction between the second substance and the reagent; and
evaluating a physical quantity of the second substance based on the change amount.

In such evaluation method, the physical quantity of the second substance can be evaluated based on the change amount of the physical quantity of the reaction products per unit time.

Now preferred embodiments of the invention will be described in detail below with reference to the drawings. It is noted that the embodiments described below do not unduly limit the contents of the invention described in the claims. Further, all of the components described below are not necessarily essential requirements of the invention.

### 1. Evaluation Method

### 1.1. Method of measuring Amount of LDL Cholesterol

First, a method of measuring the amount of LDL cholesterol in a specimen (quantitative measurement) is described. Examples of the specimen to be subjected to the measurement include: body fluids, such as blood, serum, plasma, a cerebrospinal fluid, urine, sweat, a punctured fluid, and feces; and products obtained by pretreating (e.g., diluting) these body fluids. In addition, the LDL cholesterol is cholesterol in an LDL.

The method of measuring the amount of the LDL cholesterol includes: a first step of erasing a lipoprotein except an LDL in the specimen in the presence of a first surfactant that acts on the lipoprotein except the LDL; and a second step of causing a peroxidase to act on hydrogen peroxide, which is produced by adding a second surfactant to solubilize the remaining LDL, and subjecting the resultant to an oxidation reaction with a cholesterol oxidase, to introduce the hydrogen peroxide to a color reaction, followed by the measurement of an absorbance.

In the method of measuring the amount of the LDL cholesterol, the measurement is performed with reagents for LDL cholesterol measurement. The reagents for LDL cholesterol measurement include a first reagent and a second reagent. The first reagent is, for example, an enzyme liquid containing 4-aminoantipyrine, the cholesterol oxidase, a cholesterol esterase, the peroxidase, and the first surfactant. The second reagent is, for example, a color-developing liquid containing a chromogen (N,N-bis(4-sulfobutyl)-m-toluidine (DSBmT)) and the second surfactant. The first reagent is added to the specimen in the first step, and the second reagent is added to the specimen in the second step. The cholesterol oxidase is not particularly limited as long as the oxidase is an enzyme having an ability to oxidize cholesterol to produce hydrogen peroxide. In addition, the cholesterol esterase is not particularly limited as long as the esterase is an enzyme having an ability to hydrolyze cholesterol.

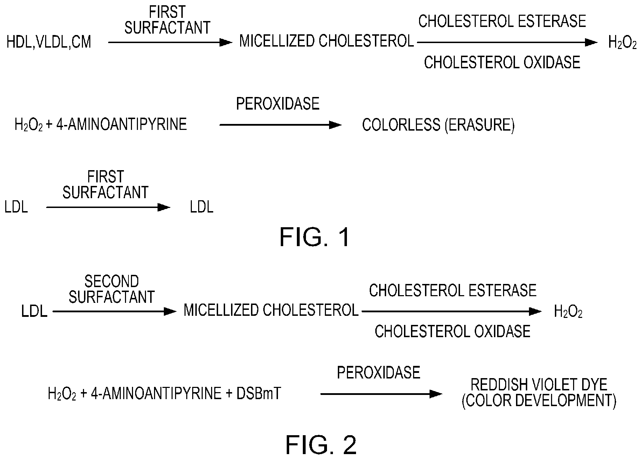

FIG. 1 is a diagram for describing the first step of the method of measuring the amount of the LDL cholesterol.

In the first step, the cholesterol esterase and the cholesterol oxidase are caused to act on cholesterol in the lipoprotein except the LDL (e.g., a high-density lipoprotein (HDL), a very low-density lipoprotein (VLDL), or a chylomicron (CM)) in the presence of the first surfactant to produce hydrogen peroxide. Each of the enzymes to be used herein may be unmodified, or may be modified with a group containing polyethylene glycol, polypropylene glycol, or the like as a main component, a group having a copolymer of the polypropylene glycol and the polyethylene glycol, a group containing sugar in its structure, a sulfopropyl group, or a polyurethane group. The first surfactant dissolves the external wall of the lipoprotein except the LDL to elute cholesterol in the lipoprotein except the LDL. Thus, cholesterol in the lipoprotein except the LDL can be introduced to an enzymatic reaction using each of the cholesterol esterase and the cholesterol oxidase. The produced hydrogen peroxide is erased by the peroxidase and 4-aminoantipyrine. The term "erased" means that the hydrogen peroxide is made colorless so as not to be detected in the absorbance measurement in the second step.

The first surfactant has an action of changing only the structure of the lipoprotein except the LDL, and does not change the structure of the LDL. That is, the first surfactant does not dissolve the external wall of the LDL. The first surfactant is, for example, a polyalkylene oxide derivative having an HLB value of 13 or more and 15 or less. Examples of the derivative include a higher alcohol condensate, a higher fatty acid condensate, a higher fatty acid amide condensate, a higher alkylamine condensate, a higher alkylmercaptan condensate, and an alkylphenol condensate. Examples of the polyalkylene oxide derivative having an HLB value of 13 or more and 15 or less include, but not limited to, compounds each having an HLB value of 13 or more and 15 or less including: a polyoxyethylene alkyl ether, such as a polyoxyethylene lauryl ether, a polyoxyethylene cetyl ether, a polyoxyethylene oleyl ether, or a polyoxyethylene isodecyl ether; a polyoxyethylene alkyl phenyl ether, such as a polyoxyethylene octyl phenyl ether or a polyoxyethylene nonyl phenyl ether; and a polyoxyethylene tribenzyl phenyl ether. The first surfactant may be a surfactant having a branched alkyl ether structure. The first surfactant may be a surfactant having no double bond such as a polyoxyethylene polyoxypropylene alkyl ether. The first surfactant may be a combination of a polyoxyethylene polyoxypropylene copolymer and a polyglyceryl ether.

Examples of a commercially available product of the first surfactant include EMULGEN B-66 (polyoxyethylene tribenzyl phenyl ether, HLB=13.2) manufactured by Kao Corporation and SAFETYCUT ID-1087 (polyoxyethylene isodecyl ether) manufactured by AOKI OIL INDUSTRIAL Co., Ltd.

In addition, a cationic surfactant may be used as the first surfactant. The first surfactant may be a polymer compound having an allylamine or diallylamine unit. The first surfactant may be a cholic acid derivative or a salt thereof. In the first surfactant, in order to reduce its reaction with the LDL to further enhance the erasure of any other lipoprotein, a divalent metal ion may be incorporated into a reaction liquid. A copper ion, an iron ion, and a magnesium ion may each be used as the divalent metal ion. Of those, a magnesium ion is particularly preferred. A lipoprotein-degrading enzyme may be optionally incorporated into the reaction liquid of the first step. The addition of the enzyme is preferred because the addition facilitates, in particular, the reaction of cholesterol in a VLDL with each of the cholesterol esterase and the cholesterol oxidase.

FIG. 2 is a diagram for describing the second step of the method of measuring the amount of the LDL cholesterol.

In the second step, the second reagent is added to the specimen that has been subjected to the first step. The second surfactant in the second reagent dissolves the external wall of the LDL remaining in the first step to elute cholesterol in the LDL. The cholesterol esterase and the cholesterol oxidase are caused to act on the eluted cholesterol to produce hydrogen peroxide. In the second step, a toluidine-based dye precursor such as DSBmT or an aromatic dye precursor similar thereto is added as a color-developing agent to introduce the produced hydrogen peroxide to the color reaction. In the color reaction, a peroxide-reducing enzyme is caused to act on the hydrogen peroxide to subject an aromatic nucleophile and an aromatic electrophile serving as dye precursors to an oxidative bonding reaction, to thereby produce a dye. Specifically, the peroxidase is caused to act on the hydrogen peroxide to subject 4-aminoantipyrine and DSBmT to an oxidative bonding reaction, to thereby produce a reddish violet dye. The amount of the LDL cholesterol is determined by measuring an absorbance for the color development of the reddish violet dye.

The second surfactant may have an action of changing the structures of all lipoproteins, or may have an action of changing only the structure of the LDL. The second surfactant having an action of changing the structures of all the lipoproteins is, for example, a polyalkylene oxide derivative having an HLB value of 11 or more and less than 13. Examples of the derivative include a higher alcohol condensate, a higher fatty acid condensate, a higher fatty acid amide condensate, a higher alkylamine condensate, a higher alkylmercaptan condensate, and an alkylphenol condensate.

Examples of the polyalkylene oxide derivative having an HLB value of 11 or more and less than 13 include, but not limited to, compounds each having an HLB value of 11 or more and less than 13, such as a polyoxyethylene lauryl ether, a polyoxyethylene cetyl ether, a polyoxyethylene oleyl ether, a polyoxyethylene higher alcohol ether, a polyoxyethylene octyl phenyl ether, and polyoxyethylene nonyl phenyl ether.

Examples of a commercially available product of the second surfactant include EMULGEN A-60 (polyoxyethylene distyrenated phenyl ether, HLB=12.8) manufactured by Kao Corporation and Triton (trademark) X-100.

In addition, the second surfactant having an action of changing only the structure of the LDL is, for example, an anionic surfactant. The anionic surfactant is preferably a surfactant having a structure in which a linear or branched alkyl group having 4 to 18 carbon atoms is bonded to an aromatic ring. Herein, the aromatic ring is preferably an aromatic ring formed only of carbon and hydrogen, such as benzene, naphthalene, or diphenyl. Further, a hydrophilic group such as a sulfonate is preferably bonded to the above-mentioned aromatic ring.

### 1.2. Method of evaluating Amount of Oxidized LDL

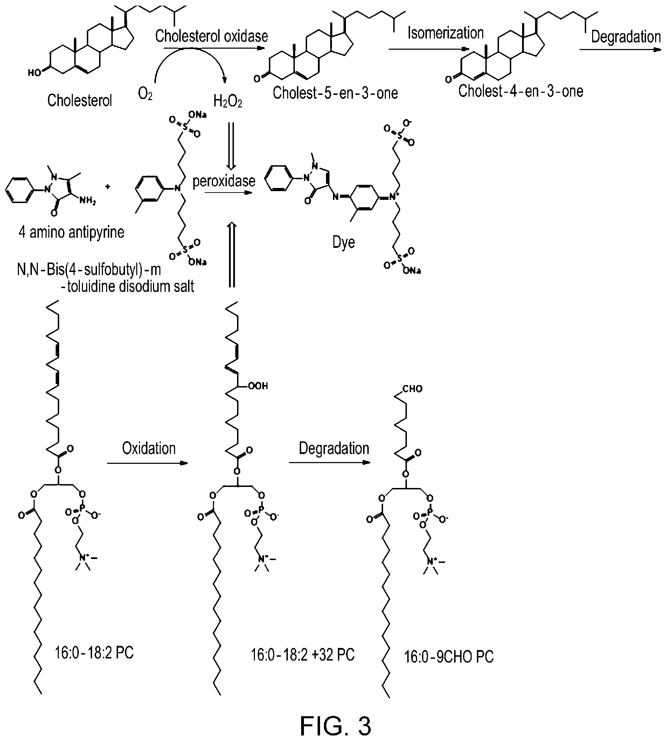

Next, a method of evaluating the amount of an oxidized LDL according to an embodiment of the invention is described. FIG. 3 is a diagram for describing the method of evaluating the amount of the oxidized LDL. The term "oxidized LDL" refers to a product obtained through the oxidative modification of an LDL by the action of active oxygen or the like. The amount of the oxidized LDL serves as an important indicator of a disease resulting from a change in function of a vascular endothelial cell, such as arteriosclerosis or a coronary syndrome.

In the second step, as illustrated in FIG. 3, when the cholesterol oxidase is caused to react with cholesterol, a hydroxy group at 3-position of cholesterol is oxidized to provide a carbonyl group. Further, the carbonyl group causes transition to advance a degradation reaction. As a result, cholesterol is erased.

Hydrogen peroxide is produced along with the oxidation of cholesterol by the cholesterol oxidase described above. When the produced hydrogen peroxide is used as a substrate, and its peroxide structure is cleaved by the peroxidase, two protons are captured from 4-aminoantipyrine and the toluidine compound (DSBmT), and both the compounds are oxidatively bonded to each other. Thus, the dye is produced.

Herein, as in the hydrogen peroxide, a lipid peroxide (16:O-18:2+32PC) produced through the oxidation of a phospholipid by active oxygen has a peroxide structure, and hence serves as a substrate for the peroxidase. Accordingly, as in the hydrogen peroxide, when the peroxide structure of the lipid peroxide is cleaved by the peroxidase, two protons are captured from 4-aminoantipyrine and the toluidine compound (DSBmT), and both the compounds are oxidatively bonded to each other to produce the dye. In addition, as in the case of the above-mentioned LDL cholesterol, the lipid peroxide undergoes a reaction with the peroxide to be degraded.

Accordingly, when the main reaction of the reagent is defined as a color reaction based on a reaction between the LDL and the reagent, and the side reaction of the reagent is defined as a color reaction based on a reaction between the oxidized LDL and the reagent, the amount of the lipid peroxide (oxidized LDL) can be evaluated by observing the side reaction. In this embodiment, the side reaction is observed while attention is paid to the fact that the side reaction is delayed with respect to the main reaction.

One cause of the delay of the side reaction with respect to the main reaction is a difference between the rate of a solubilization reaction at the time of the elution of cholesterol through the solubilization of the LDL with the second surfactant and the rate of a solubilization reaction at the time of the elution of the lipid peroxide through the solubilization of the oxidized LDL with the second surfactant. That is, the rate of the solubilization reaction at the time of the elution of the lipid peroxide through the solubilization of the oxidized LDL is slower than the rate of the solubilization reaction at the time of the elution of cholesterol through the solubilization of the LDL. The difference between the rates of the solubilization reactions may be due to a change in ability of the second surfactant to dissolve the external wall of the LDL by the oxidative modification of the external wall of the LDL.

In addition, another cause of the delay of the side reaction with respect to the main reaction is a difference in reaction rate between the hydrogen peroxide and the lipid peroxide serving as substrates for the peroxidase. The side reaction is delayed with respect to the main reaction because the reaction rate of the lipid peroxide with respect to the peroxidase is slower than the reaction rate of the hydrogen peroxide with respect to the peroxidase.

When the LDL is oxidatively modified, various phenomena, such as the advance of a lipid peroxidation reaction, the modification of an apoprotein, and a change in particle state, occur. Accordingly, the delay of the side reaction with respect to the main reaction may be caused not only by the above-mentioned two elements but also by the overlap of various elements.

An oxidized phospholipid (OxPL) is detected by apolipoprotein B (apoB) in plasma, and an OxPL-apoB shows a stronger correlation with lipoprotein (a) [Lp(a)] that is one subtype of a lipoprotein. That is, the foregoing shows that Lp(a) is the best carrier of the OxPL, and hence its involvement in the delay of the side reaction with respect to the main reaction is suggested.

An LDL-C value measured with a general reagent for a clinical test is the sum of true LDL-C and cholesterol in Lp(a) (LDL(a)-C). A method including extracting LDL(a) with an anti-LDL(a) antibody bound to magnetic beads and determining its amount has been known as a method of measuring the amount of the LDL(a)-C. When the reaction delay of the side reaction represents an LDL(a)-C value or some involvement in the delay is found, this embodiment is useful because there is no need to perform such complicated operation.

In addition, the LDL is divided into finer subfractions in accordance with lipid composition, and the subfractions are more easily oxidized as their particle diameters become smaller. The small particles have a heavy specific gravity, and are called a small dense LDL (sd-LDL). Large particles have a light specific gravity, and are called a large buoyant LDL (lb-LDL). It has been pointed out that the sd-LDL out of the LDL particles has higher arteriosclerosis inducibility. Although both the sd-LDL and the lb-LDL show positive correlations with an LDL-C value, the sd-LDL and the lb-LDL show a negative correlation with each other. The reaction delay of the side reaction may be derived from a component for forming the sd-LDL.

Such delay of the side reaction with respect to the main reaction may be observed by, for example, measuring the change amount of the absorbance per unit time in the above-mentioned method of measuring the amount of the LDL cholesterol. In this embodiment, the amount of the oxidized LDL is evaluated based on the change amount of the absorbance per unit time.

### 2. Experimental Example

A method of evaluating the amount of an oxidized LDL is specifically described below by way of Experimental Examples, but the invention is not limited to these Examples.

### 2.1. Measurement Method

Measuring apparatus: JCA-BM8040 manufactured by JEOL Ltd.
Reagent: CHOLESTEST LDL manufactured by Sekisui Medical Co., Ltd.

### <First Reagent (Enzyme Liquid)>

The liquid contains 4-aminoantipyrine, a cholesterol oxidase, a cholesterol esterase, a peroxidase, and a first surfactant (EMULGEN B-66).

### <Second Reagent (Color-developing Liquid)>

The liquid contains DSBmT and a second surfactant (EMULGEN A-60).

### (1) Quantitative Measurement of LDL Cholesterol

60 Microliters of the first reagent is added to 3.0 µL of a serum specimen diluted five-fold with physiological saline, and the mixture is incubated at 37°C for 5 minutes. Before the addition of the second reagent, the mixture is subjected to photometry at two wavelengths, that is, a main wavelength of 545 nm and a sub-wavelength of 658 nm. The results are defined as first absorbance data.

Next, 20 µL of the second reagent is further added to the mixture, and the whole is incubated at 37°C for 5 minutes, followed by photometry at two wavelengths, that is, a main wavelength of 545 nm and a sub-wavelength of 658 nm. The results are defined as second absorbance data. To correct the color tone of the specimen, the first absorbance data is subtracted from the second absorbance data. The first absorbance data to be subtracted is an absorbance after the performance of liquid amount correction.

The LDL cholesterol concentration of the serum specimen is determined from the absorbance of a standard solution measured by the same procedure as that of the specimen described above.

### (2) Differential Measurement

To measure the change amount of an absorbance per unit time, differential measurement is performed simultaneously with the above-mentioned quantitative measurement of the LDL cholesterol. Specifically, after the addition of the second reagent, rate photometry (differential measurement) is performed at two wavelengths, that is, a main wavelength of 545 nm and a sub-wavelength of 658 nm during a period from a time point 3 minutes before the endpoint of a reaction between the cholesterol and the reagent to the endpoint of the reaction (during a period from 45 points (415 seconds) to 64 points (598 seconds)), and the change amount of the absorbance per 1 minute is determined. A value obtained by multiplying the change amount of the absorbance per 1 minute during the period from the time point 3 minutes before the endpoint of the reaction to the endpoint of the reaction by a constant of 10,000 is defined as a reaction delay indicator.

### 2.2. Experimental Example 1

### < Specimen >

0.02 Milliliter of a copper sulfate solution (CLINIMATE TP measuring reagent manufactured by Sekisui Medical Co., Ltd.) was added to 0.5 mL of volunteer serum, and the mixture was stirred by inversion. After that, the mixture was incubated at 50°C for 20 days so that an LDL in the serum was oxidized. Thus, an oxidation-treated specimen was produced.

In addition, 0.02 mL of ion-exchanged water was added to 0.5 mL of volunteer serum, and the mixture was stirred by inversion. After that, the mixture was incubated at 50°C for 20 days to produce a control specimen.

### <Measurement>

The oxidation-treated specimen and the control specimen were each subjected to the above-mentioned quantitative measurement of LDL cholesterol. In addition, the oxidation-treated specimen and the control specimen were each subjected to the above-mentioned differential measurement, and the change amount of the absorbance of each of the specimens per 1 minute was determined, followed by the calculation of a reaction delay indicator from the change amount.

### <Measurement Result>

### (1) Result of Quantitative Measurement of LDL Cholesterol

The LDL cholesterol concentration of the oxidation-treated specimen was 131 mg/dL. In addition, the LDL cholesterol concentration of the control specimen was 138 mg/dL.

### (2) Result of Differential Measurement

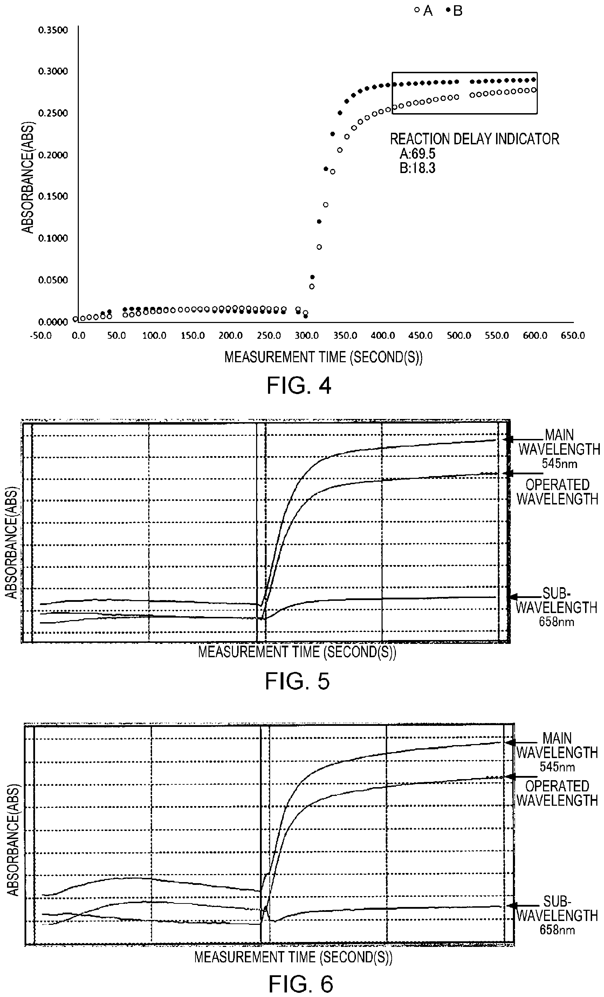

FIG. 4 is a graph illustrating results of the differential measurement of the oxidation-treated specimen. The axis of abscissa of the graph in FIG. 4 indicates a measurement time (second(s)), and the axis of ordinate thereof indicates an absorbance (ABS). In addition, the graph in FIG. 4 illustrates results A of the measurement of the oxidation-treated specimen, and results B of the measurement of the control specimen.

As illustrated in FIG. 4, the differential analysis of an absorbance curve showing a change in absorbance during a period from a time point 3 minutes before the endpoint of a reaction between the LDL and the reagent to the endpoint of the reaction was performed to calculate the change amount of the absorbance of the oxidation-treated specimen per 1 minute. The change amount (ΔABS/min) of the absorbance of the control specimen per 1 minute was similarly calculated. A value (ΔABS/min×10,000) obtained by multiplying the change amount (ΔABS/min) of the absorbance per 1 minute by a constant of 10,000 was defined as a reaction delay indicator. The reaction delay indicator of the oxidation-treated specimen was 69.5, and the reaction delay indicator of the control specimen was 18.3. That is, the reaction delay indicator of the oxidation-treated specimen was 3.8 times as large as the reaction delay indicator of the control specimen.

As described above, in the oxidation-treated specimen, apparent reaction delay was observed as compared to the control specimen that was not subjected to any oxidation treatment.

### (3) Influence of Copper Sulfate on Reagent

The copper sulfate solution was removed from the oxidation-treated specimen by loading the oxidation-treated specimen into a dialysis tube and performing dialysis. The oxidation-treated specimen after the dialysis and the oxidation-treated specimen before the dialysis were each subjected to differential measurement.

FIG. 5 is a graph illustrating results of the measurement of the absorbance of the oxidation-treated specimen before the dialysis. FIG. 6 is a graph illustrating results of the measurement of the absorbance of the oxidation-treated specimen after the dialysis. The graphs in FIG. 5 and FIG. 6 each illustrate a main wavelength absorbance, a sub-wavelength absorbance, and an operated absorbance obtained by subtracting the sub-wavelength absorbance from the main wavelength absorbance.

Reaction delay in the oxidation-treated specimen after the dialysis was able to be recognized from the graph in FIG. 5 and the graph in FIG. 6. That is, it was able to be recognized that the reaction delay of the oxidation-treated specimen was not reaction delay due to absorption derived from a copper ion.

### 2.3. Experimental Example 2

### < Specimen >

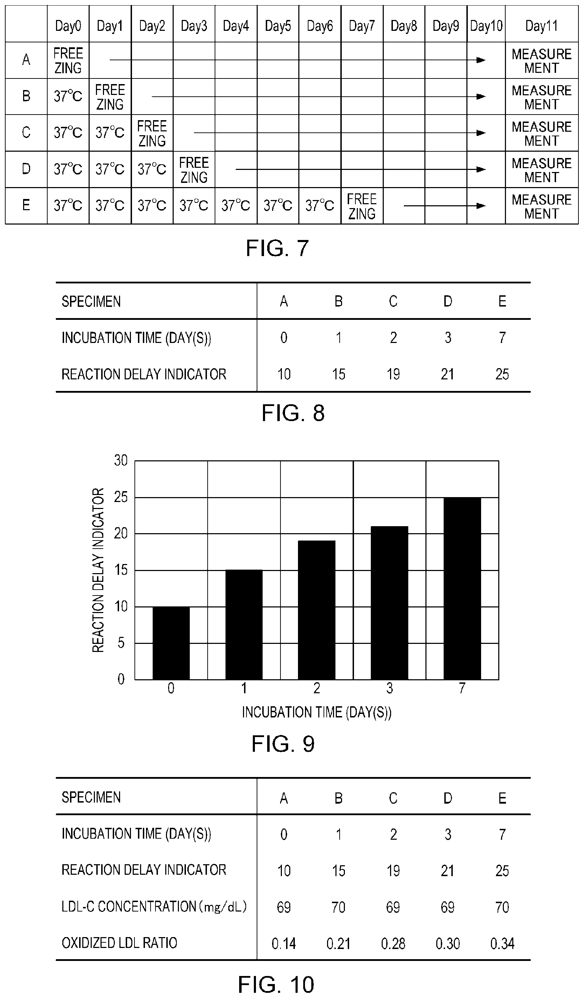

FIG. 7 is a table illustrating conditions for the production of oxidation-treated specimens A, B, C, D, and E. 0.02 Milliliter (final concentration: 3.8%) of a copper sulfate solution (CLINIMATE TP measuring reagent manufactured by Sekisui Medical Co., Ltd.) was added to 0.5 mL of base serum to produce the oxidation-treated specimen A. That is, the incubation time of the oxidation-treated specimen A is 0 days. In addition, 0.02 mL (final concentration: 3.8%) of a copper sulfate solution (CLINIMATE TP measuring reagent manufactured by Sekisui Medical Co., Ltd.) was added to 0.5 mL of base serum, and the mixture was incubated at 37°C for 1 day to produce the oxidation-treated specimen B. The oxidation-treated specimen C, the oxidation-treated specimen D, and the oxidation-treated specimen E were produced in the same manner as in the oxidation-treated specimen B while the incubation time was changed to 2 days, 3 days, and 7 days, respectively.

### <Measurement>

The oxidation-treated specimen A was subjected to differential measurement, and the change amount (ΔABS/min) of the absorbance thereof per 1 minute was determined, followed by the calculation of the reaction delay indicator (ΔABS/min×10,000) thereof from the change amount. The reaction delay indicator (ΔABS/min×10,000) of each of the oxidation-treated specimens B, C, D, and E was calculated in the same manner as in the oxidation-treated specimen A.

### <Measurement Result>

FIG. 8 is a table illustrating the incubation times (oxidation treatment times) and reaction delay indicators of the oxidation-treated specimens. FIG. 9 is a histogram illustrating the reaction delay indicator for each incubation time.

As illustrated in each of FIG. 8 and FIG. 9, it was found that the reaction delay indicator became larger as the oxidation treatment time for the serum lengthened. That is, it was found that an oxidized LDL amount was able to be evaluated by the reaction delay indicator.

In addition, the oxidized LDL amount of an actual specimen can be evaluated by comparing the reaction delay indicator of the actual specimen to the results illustrated in FIG. 8 and FIG. 9. For example, the oxidized LDL amount of the actual specimen may be evaluated by examining where the reaction delay indicator of the actual specimen is positioned in a range determined as follows: in the range, the reaction delay indicator of the oxidation-treated specimen A whose oxidation treatment time is 0 days is defined as the minimum value of the evaluation value of the oxidized LDL amount, and the reaction delay indicator of the oxidation-treated specimen E whose oxidation treatment time is 7 days is defined as the maximum value of the evaluation value of the oxidized LDL amount.

### 2.4. Experimental Example 3

### <Specimen>

An oxidation-treated specimen A, an oxidation-treated specimen B, an oxidation-treated specimen C, an oxidation-treated specimen D, and an oxidation-treated specimen E were produced in the same manner as in Experimental Example 2.

### <Measurement>

The oxidation-treated specimen A was subjected to the quantitative measurement of LDL cholesterol, and the LDL cholesterol concentration (LDL-C concentration) thereof was determined. In addition, the oxidation-treated specimen A was subjected to differential measurement, and the reaction delay indicator (ΔABS/min×10,000) thereof was calculated. Further, an oxidized LDL ratio ((ΔABS/min×10,000)/LDL-C concentration) was calculated by dividing the reaction delay indicator (ΔABS/min×10,000) of the oxidation-treated specimen A by the LDL cholesterol concentration (LDL-C concentration). The oxidized LDL ratio was used as the evaluation value of the oxidized LDL amount of the specimen.

The reaction delay indicators and LDL cholesterol concentrations of the oxidation-treated specimens B, C, D, and E, and the evaluation values of the oxidized LDL amounts thereof were determined in the same manner as in the oxidation-treated specimen A.

### <Measurement Result>

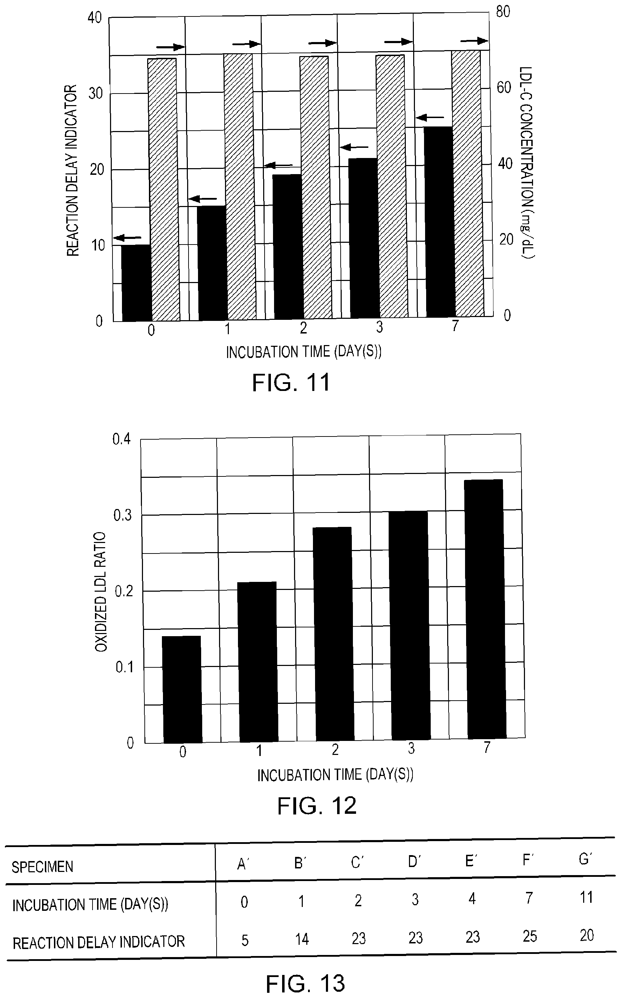

FIG. 10 is a table illustrating the incubation times, reaction delay indicators, LDL cholesterol concentrations, and oxidized LDL ratios of the respective oxidation-treated specimens. FIG. 11 is a histogram illustrating the reaction delay indicator and the LDL cholesterol concentration for each incubation time. FIG. 12 is a histogram illustrating the oxidized LDL ratio for each incubation time.

In each of the oxidized LDL ratios, the reaction delay indicator is normalized by the concentration of the LDL cholesterol in the specimen, and hence the oxidized LDL amounts can be compared to each other even between the specimens having different LDL cholesterol concentrations. Accordingly, in the case where the oxidized LDL ratios are used as the evaluation values of the oxidized LDL amounts, even between the specimens having different LDL cholesterol concentrations, their oxidized LDL amounts can be more accurately evaluated as compared to the case where the reaction delay indicators are used as the evaluation values of the oxidized LDL amounts.

### 2.5. Experimental Example 4

### < Specimen >

0.05 Milliliter (final concentration: 9.1%) of a copper sulfate solution (CLINIMATE TP measuring reagent manufactured by Sekisui Medical Co., Ltd.) was added to 0.5 mL of base serum to produce an oxidation-treated specimen A'. In addition, 0.05 mL (final concentration: 9.1%) of a copper sulfate solution (CLINIMATE TP measuring reagent manufactured by Sekisui Medical Co., Ltd.) was added to 0.5 mL of base serum, and the mixture was incubated at 50°C for 1 day to produce an oxidation-treated specimen B'. An oxidation-treated specimen C', an oxidation-treated specimen D', an oxidation-treated specimen E', an oxidation-treated specimen F', and an oxidation-treated specimen G' were produced in the same manner as in the oxidation-treated specimen B' while the incubation time was changed to 2 days, 3 days, 4 days, 7 days, and 11 days, respectively.

### <Measurement>

The oxidation-treated specimen A' was subjected to differential measurement, and the change amount (ΔABS/min) of the absorbance thereof per 1 minute was determined, followed by the calculation of the reaction delay indicator (ΔABS/min×10,000) thereof from the change amount. The reaction delay indicator (ΔABS/min×10,000) of each of the oxidation-treated specimens B', C', D', E', F', and G' was calculated in the same manner.

### <Measurement Result>

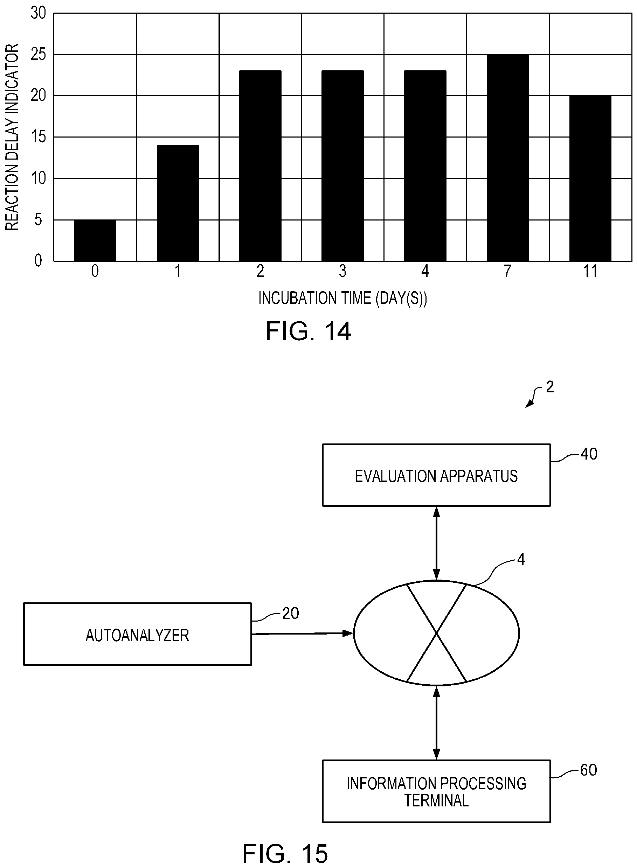

FIG. 13 is a table illustrating the incubation times and reaction delay indicators of the oxidation-treated specimens. FIG. 14 is a histogram illustrating the reaction delay indicator for each incubation time.

As illustrated in each of FIG. 13 and FIG. 14, the reaction delay indicator was saturated at an oxidation treatment time of 2 days. This is probably because of the following reason: in the conditions for the production of the oxidation-treated specimens of Experimental Example 4, the final concentration of the copper sulfate solution was high and an incubation temperature was high as compared to those in the conditions for the production of the oxidation-treated specimens of Experimental Example 2, and hence under the conditions for the production of the oxidation-treated specimens of Experimental Example 4, an oxidized LDL was produced in an amount larger than that under the conditions for the production of the oxidation-treated specimens of Experimental Example 2.

### 3. System

### 3.1. Configuration of System

An evaluation system according to an embodiment of the invention is described with reference to the drawings. FIG. 15 is a diagram for illustrating a configuration of an evaluation system 2 according to an embodiment of the invention.

As illustrated in FIG. 15, the evaluation system 2 includes an autoanalyzer 20, an evaluation apparatus 40, and an information processing terminal 60.

As illustrated in FIG. 15, the evaluation system 2 is configured so that the autoanalyzer 20, the evaluation apparatus 40, and the information processing terminal 60 can be connected to each other through a communication network 4 such as the Internet. The communication network 4 is not limited to the Internet, and may be a local area network (LAN), a wide area network (WAN), or the like.

The autoanalyzer 20 is a biochemical autoanalyzer that automatically measures the amount of a specific component in a specimen. The autoanalyzer 20 can measure an LDL cholesterol concentration through the above-mentioned quantitative measurement of LDL cholesterol. Further, the autoanalyzer 20 can measure the change amount of an absorbance per unit time through the above-mentioned differential measurement.

The autoanalyzer 20 transmits information on the LDL cholesterol concentration and information on the change amount of the absorbance per unit time to the evaluation apparatus 40 through the communication network 4. For example, when the autoanalyzer 20 transmits the information on the LDL cholesterol concentration and the information on the change amount of the absorbance per unit time to the evaluation apparatus 40 through the communication network 4, and requires the evaluation apparatus 40 to perform evaluation processing of an oxidized LDL amount, the evaluation apparatus 40 performs processing for evaluating the oxidized LDL amount.

The evaluation apparatus 40 acquires the information on the LDL cholesterol concentration and the information on the change amount of the absorbance per unit time from the autoanalyzer 20 through the communication network 4. The evaluation apparatus 40 evaluates the amount of an oxidized LDL in the specimen based on the change amount of the absorbance per unit time. In addition, the evaluation apparatus 40 evaluates the amount of the oxidized LDL in the specimen based on the LDL cholesterol concentration and the change amount of the absorbance per unit time. The evaluation apparatus 40 transmits the result of the evaluation of the oxidized LDL amount to the information processing terminal 60 through the communication network 4.

The information processing terminal 60 acquires the result of the evaluation of the oxidized LDL amount from the evaluation apparatus 40 through the communication network 4. When a user accesses the evaluation apparatus 40 from the information processing terminal 60, the evaluation result can be acquired from the evaluation apparatus 40 through the communication network 4. The evaluation result is displayed on a displaying unit of the information processing terminal 60. The information processing terminal 60 is, for example, a portable terminal such as a smartphone or an information processing apparatus such as a personal computer (PC).

The evaluation system 2 may be a cloud-type evaluation system. In, for example, the cloud-type evaluation system 2, when the autoanalyzer 20 requires the evaluation apparatus 40 to perform processing for evaluating the oxidized LDL amount, and transmits the pieces of information on the measurement results thereto, the evaluation apparatus 40 serving as a server apparatus performs the processing for evaluating the oxidized LDL amount. In addition, the information processing terminal 60 acquires the result of the evaluation processing (the result of the evaluation of the oxidized LDL amount) in the evaluation apparatus 40 from the evaluation apparatus 40, and performs such control that the result of the evaluation processing is displayed on the displaying unit.

### 3.2. Autoanalyzer

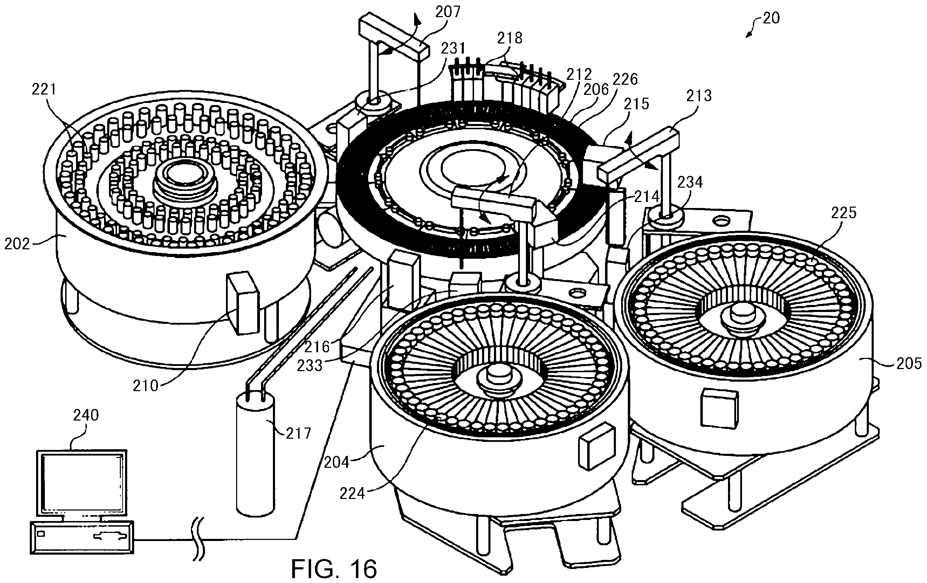

FIG. 16 is a view for schematically illustrating an example of a configuration of the autoanalyzer 20.

The autoanalyzer 20 includes: a sample turntable 202; a first turntable 204; a second turntable 205; a reaction turntable 206; a specimen-dispensing probe 207; a sample barcode reader 210; a first reagent-dispensing probe 212; a second reagent-dispensing probe 213; a first reaction liquid-stirring mechanism 214; a second reaction liquid-stirring mechanism 215; a multiwavelength photometer 216; a thermostat 217; a reaction container-washing mechanism 218; a specimen-dispensing probe-washing mechanism 231; a first reagent-dispensing probe-washing mechanism 233; a second reagent-dispensing probe-washing mechanism 234; and a controlling unit 240.

Each of the sample turntable 202, the first turntable 204, the second turntable 205, and the reaction turntable 206 is rotatably supported along its circumferential direction by a driving mechanism (not shown), and rotates in the circumferential direction for each predetermined angle range at a predetermined speed.

The sample turntable 202 holds a plurality of sample containers 221. The specimen is stored in each of the sample containers 221.

The first turntable 204 holds a plurality of first reagent containers 224, and the second turntable 205 holds a plurality of second reagent containers 225. A first reagent is stored in each of the first reagent containers 224, and a second reagent is stored in each of the second reagent containers 225.

The reaction turntable 206 holds a plurality of reaction containers 226. The plurality of reaction containers 226 are stored in line with the circumferential direction of the reaction turntable 206, and the reaction turntable 206 intermittently moves the reaction containers 226 in the circumferential direction. The specimen sampled from each of the sample containers 221, the first reagent sampled from each of the first reagent containers 224, and the second reagent sampled from each of the second reagent containers 225 are injected into each of the reaction containers 226. In the reaction container 226, the specimen, the first reagent, and the second reagent are stirred to perform a reaction.

The specimen-dispensing probe 207 sucks a predetermined amount of the specimen from the sample container 221 conveyed to a preset sucking position, and ejects the sucked specimen into the reaction container 226 conveyed to a preset ejecting position. The specimen-dispensing probe 207 is washed by the specimen-dispensing probe-washing mechanism 231.

The sample barcode reader 210 reads a specimen ID from a barcode attached to the side surface of each of the sample containers 221 stored in the sample turntable 202. The identification information read by the sample barcode reader 210 is transmitted to the controlling unit 240. Thus, in the controlling unit 240, the specimen stored in each of the sample containers 221 and dispensed into each of the reaction containers 226 by the specimen-dispensing probe 207 can be managed.

The first reagent-dispensing probe 212 sucks a predetermined amount of the first reagent from the first reagent container 224 conveyed to a preset sucking position, and ejects the sucked first reagent into the reaction container 226 conveyed to a preset ejecting position. The first reagent-dispensing probe 212 is washed by the first reagent-dispensing probe-washing mechanism 233.

The second reagent-dispensing probe 213 sucks a predetermined amount of the second reagent from the second reagent container 225 conveyed to a preset sucking position, and ejects the sucked second reagent into the reaction container 226 conveyed to a preset ejecting position. The second reagent-dispensing probe 213 is washed by the second reagent-dispensing probe-washing mechanism 234.

The first reaction liquid-stirring mechanism 214 inserts a stirring rod (not shown) into each of the reaction containers 226, and stirs the mixed liquid of the specimen and the first reagent in the reaction container 226. The second reaction liquid-stirring mechanism 215 inserts a stirring rod (not shown) into each of the reaction containers 226, and stirs the mixed liquid of the specimen, the first reagent, and the second reagent in the reaction container 226. The reaction container-washing mechanism 218 washes the inside of the reaction container 226 whose analysis has been completed.

The multiwavelength photometer 216 performs optical measurement (colorimetric determination) on the mixed liquid of the specimen that has reacted with the first reagent and the second reagent through use of a light source lamp that applies light beams to the reaction containers 226. The multiwavelength photometer 216 outputs the amounts of various components in the specimen as absorbances, and detects the reaction state of the specimen. Information on the result of the measurement in the multiwavelength photometer 216 is transmitted to the controlling unit 240. The thermostat 217 holds the temperatures of the reaction containers 226 stored in the reaction turntable 206 constant at all times.

The controlling unit 240 acquires information on the absorbances output from the multiwavelength photometer 216, and performs various kinds of operation processing. The controlling unit 240 performs processing, such as processing for subtracting a sub-wavelength absorbance from a main wavelength absorbance to calculate an operated absorbance, differential processing for calculating the change amount of an absorbance per unit time, or processing for calculating the concentration of a substance from its absorbance. In addition, the controlling unit 240 performs processing for controlling the respective portions of the autoanalyzer 20. The controlling unit 240 includes, for example, a central processing unit (CPU) and storage devices (e.g., a random access memory (RAM) and a read only memory (ROM)). The controlling unit 240 performs various kinds of operation processing and various kinds of control processing through the execution of programs stored in the storage devices by the CPU.

In the autoanalyzer 20, the amount of LDL cholesterol can be measured with the above-mentioned reagents for LDL cholesterol measurement. In the autoanalyzer 20, the LDL cholesterol concentration may be measured by, for example, an endpoint method. Further, in the autoanalyzer 20, the change amount of the absorbance per unit time can be measured by differential measurement. The endpoint method is a method including: measuring an absorbance at the time of the completion of a reaction; and determining the concentration of a target substance in a specimen with respect to the concentration of a standard solution and the absorbance of the standard solution. In the differential measurement, absorbance measurement is repeated at certain time intervals, and the change amount of the absorbance per unit time is calculated from the resultant data. In the differential measurement, the change amount of the absorbance per unit time during a period from a time point dating back a predetermined time from the endpoint of the reaction to the endpoint of the reaction can be measured. The predetermined time and the unit time may be freely set.

The sample turntable 202, the first turntable 204, the second turntable 205, the reaction turntable 206, the specimen-dispensing probe 207, the sample barcode reader 210, the first reagent-dispensing probe 212, the second reagent-dispensing probe 213, the first reaction liquid-stirring mechanism 214, the second reaction liquid-stirring mechanism 215, the multiwavelength photometer 216, the thermostat 217, the reaction container-washing mechanism 218, the specimen-dispensing probe-washing mechanism 231, the first reagent-dispensing probe-washing mechanism 233, and the second reagent-dispensing probe-washing mechanism 234 described above each function as a measuring unit.

In addition, the controlling unit 240 functions as a differential operation unit configured to calculate the change amount of the absorbance per unit time from the change of the absorbance with time.

### 3.3. Evaluation Apparatus

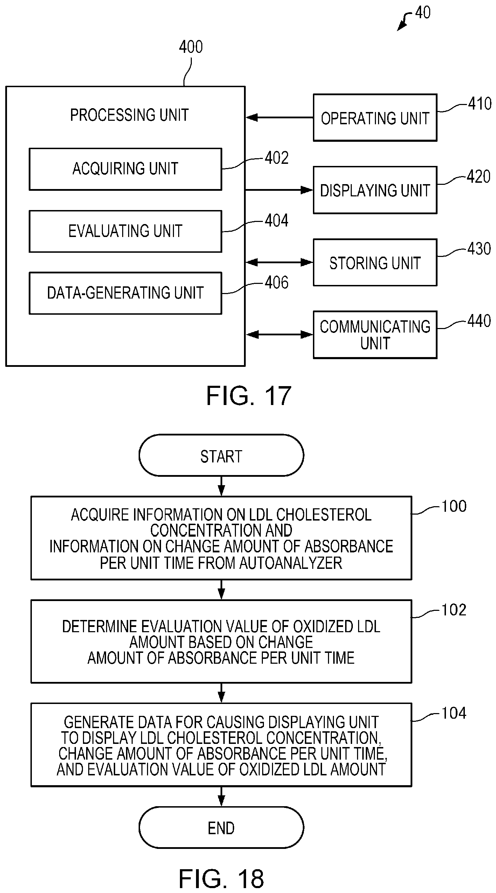

FIG. 17 is a diagram for illustrating an example of a configuration of the evaluation apparatus 40.

As illustrated in FIG. 17, the evaluation apparatus 40 includes a processing unit 400, an operating unit 410, a displaying unit 420, a storing unit 430, and a communicating unit 440.

The operating unit 410 is intended for the input of operation information by a user, and outputs the input operation information to the processing unit 400. The function of the operating unit 410 may be achieved with an input device, such as a keyboard, a mouse, a button, a touch panel, or a touch pad.

The displaying unit 420 displays an image produced by the processing unit 400, and its function may be achieved with a display, such as a liquid crystal display (LCD) or a cathode ray tube (CRT).

The storing unit 430 stores programs and various pieces of data for causing a computer to function as the respective portions of the processing unit 400. In addition, the storing unit 430 functions as a working area for the processing unit 400 and the communicating unit 440. The functions of the storing unit 430 may be achieved with a hard disk drive, a random access memory (RAM), or the like.

The communicating unit 440 performs various kinds of control for performing communication to and from the autoanalyzer 20 or the information processing terminal 60, and its function may be achieved through the execution of programs by hardware, such as various processors (e.g., a CPU and a DSP) and an ASIC for communication.

The processing unit 400 functions as an acquiring unit 402, an evaluating unit 404, and a data-generating unit 406 to be described below through the execution of the programs stored in the storing unit 430. The functions of the processing unit 400 may be achieved through the execution of the programs by hardware, such as various processors (e.g., a CPU and a DSP) and an ASIC (e.g., a gate array). The processing unit 400 includes the acquiring unit 402, the evaluating unit 404, and the data-generating unit 406.

The acquiring unit 402 acquires the pieces of information on the measurement results output from the autoanalyzer 20. The acquiring unit 402 acquires, for example, the information on the LDL cholesterol concentration and the information on the change amount of the absorbance per unit time.

The evaluating unit 404 evaluates the amount of the oxidized LDL in the specimen based on the change amount of the absorbance per unit time. The evaluating unit 404 determines the evaluation value of the oxidized LDL amount based on a value obtained by multiplying the change amount of the absorbance per unit time by a constant. For example, the evaluating unit 404 uses a value (reaction delay indicator), which is obtained by multiplying the change amount of the absorbance per 1 minute during a period from a time point 3 minutes before the endpoint of a reaction between the specimen and the reagents to the endpoint of the reaction by a constant of 10,000, as an evaluation value.

Herein, the period from the time point 3 minutes before the endpoint of the reaction to the endpoint of the reaction has been an evaluation object, but a time period serving as an evaluation object may be appropriately changed. In addition, the unit time has been set to 1 minute, but the time may be appropriately changed. In addition, the constant is not limited to 10,000, and may be set to any number.

In addition, for example, the evaluating unit 404 evaluates the amount of the oxidized LDL in the specimen based on a value obtained by dividing the change amount of the absorbance per unit time by the LDL cholesterol concentration of the specimen. For example, the evaluating unit 404 uses a value (oxidized LDL ratio) obtained as follows as an evaluation value: the change amount of the absorbance per 1 minute during the period from the time point 3 minutes before the endpoint of the reaction to the endpoint of the reaction is multiplied by a constant of 10,000; and the resultant value is divided by the LDL cholesterol concentration.

The evaluating unit 404 may use both the reaction delay indicator and the oxidized LDL ratio as the evaluation values of the oxidized LDL amount, or may use one of the values as an evaluation value thereof.

The data-generating unit 406 generates display data for causing the information processing terminal 60 to display the LDL cholesterol concentration of the specimen, the change amount of the absorbance per unit time, and the evaluation value of the oxidized LDL amount. For example, when a user accesses the evaluation apparatus 40 from the information processing terminal 60, the display data can be acquired through the communication network 4. Thus, the LDL cholesterol concentration, the change amount of the absorbance per unit time, and the evaluation value of the oxidized LDL amount are displayed on the displaying unit of the information processing terminal 60.

### 4. Processing of Evaluation Apparatus

FIG. 18 is a flowchart for illustrating an example of the evaluation processing of the oxidized LDL amount of the evaluation apparatus 40.

The acquiring unit 402 acquires the information on the LDL cholesterol concentration of the specimen and the information on the change amount of the absorbance per unit time output from the autoanalyzer 20 (Step 100).

In the measuring unit of the autoanalyzer 20, measurements based on an endpoint method and differential measurement are performed with the reagents for LDL cholesterol measurement, and the controlling unit 240 calculates the LDL cholesterol concentration and the change amount of the absorbance per unit time. The autoanalyzer 20 transmits the information on the LDL cholesterol concentration and the information on the change amount of the absorbance per unit time thus calculated to the evaluation apparatus 40 through the communication network 4, and requires the evaluation apparatus 40 to perform evaluation processing. The processing unit 400 starts the evaluation processing in accordance with the requirement, and the acquiring unit 402 acquires those pieces of information output from the autoanalyzer 20.

The evaluating unit 404 determines the evaluation value of the oxidized LDL amount based on the change amount of the absorbance per unit time (Step 102). The evaluating unit 404 calculates, for example, at least one of the reaction delay indicator or the oxidized LDL ratio as the evaluation value of the oxidized LDL amount.

The data-generating unit 406 generates the display data for causing the displaying unit to display the LDL cholesterol concentration, the change amount of the absorbance per unit time, and the evaluation value of the oxidized LDL amount (Step 104). After the data-generating unit 406 has generated the display data, the processing unit 400 completes the evaluation processing.

### 5. Effects

The evaluation apparatus 40 includes: the acquiring unit 402 configured to acquire information on a change amount of an absorbance per unit time, the change amount being obtained by a measurement method including causing a peroxidase to act on hydrogen peroxide, which is produced by solubilizing an LDL in a specimen with a surfactant that acts on the LDL, and subjecting the resultant to an oxidation reaction with a cholesterol oxidase, to introduce the hydrogen peroxide to a color reaction, followed by measurement of a change of the absorbance with time; and the evaluating unit 404 configured to evaluate an amount of an oxidized LDL in the specimen based on the change amount.

Thus, in the evaluation apparatus 40, the amount of the oxidized LDL in the specimen can be evaluated based on the change amount of the absorbance per unit time measurable with the autoanalyzer 20. As a result, in the evaluation apparatus 40, the amount of the oxidized LDL can be rapidly evaluated. Accordingly, a large oxidized LDL amount of the specimen can be easily evaluated with the evaluation apparatus 40.

In addition, when the amount of the oxidized LDL in the specimen is evaluated from the change amount of the absorbance per unit time, the oxidized LDL can be comprehensively observed in one measurement. In, for example, an ELISA method, the oxidized LDL can be detected by using an aldehyde as a target, but a change in function of a vascular endothelial cell, such as arteriosclerosis or a coronary syndrome, is not so simple as to be judgeable with one biomarker. In contrast, when the amount of the oxidized LDL in the specimen is evaluated from the change amount of the absorbance per unit time, the oxidative modification of the LDL can be comprehensively observed. Accordingly, an indicator that can more accurately evaluate a potential risk of arteriosclerosis or a coronary syndrome can be provided.

The evaluation apparatus 40 determines the evaluation value of the oxidized LDL amount based on a value obtained by multiplying the change amount of the absorbance per unit time by a constant. Accordingly, in the evaluation apparatus 40, an indicator that can more accurately evaluate a potential risk of arteriosclerosis or a coronary syndrome can be provided.

The evaluation apparatus 40 determines the evaluation value of the oxidized LDL amount based on a value obtained by dividing the change amount of the absorbance per unit time by the LDL cholesterol concentration of the specimen. As described above, in the evaluation apparatus 40, the change amount of the absorbance per unit time is normalized by the LDL cholesterol concentration of the specimen, and hence the oxidized LDL amounts can be more accurately compared to each other even between the specimens having different LDL cholesterol concentrations.

The evaluation apparatus 40 includes the data-generating unit 406 configured to generate data for causing the displaying unit of the information processing terminal 60 to display at least one of the LDL cholesterol concentration of the specimen, the change amount of the absorbance per unit time, or the evaluation value of the oxidized LDL amount. Accordingly, in the evaluation apparatus 40, the LDL cholesterol concentration of the specimen, the change amount of the absorbance per unit time, and the evaluation value of the oxidized LDL amount can be displayed on the displaying unit of the information processing terminal 60.

In the evaluation apparatus 40, the change amount of the absorbance per unit time is the change amount of the absorbance per unit time during a period from a time point dating back a predetermined time from the endpoint of a reaction between the specimen and a reagent to the endpoint of the reaction. Accordingly, in the evaluation apparatus 40, the delay of a side reaction with respect to a main reaction can be observed, and hence the oxidized LDL amount can be evaluated.

The evaluation system 2 includes: the measuring unit configured to cause a peroxidase to act on hydrogen peroxide, which is produced by solubilizing an LDL in a specimen with a surfactant that acts on the LDL, and subjecting the resultant to an oxidation reaction with a cholesterol oxidase, to introduce the hydrogen peroxide to a color reaction, followed by measurement of a change of an absorbance with time to output data on the change of the absorbance with time; the differential operation unit configured to determine a change amount of the absorbance per unit time from the data; and the evaluating unit 404 configured to evaluate an amount of an oxidized LDL in the specimen based on the change amount.

Thus, in the evaluation system 2, the amount of the oxidized LDL in the specimen can be evaluated based on the change amount of the absorbance per unit time measurable with the autoanalyzer 20. Accordingly, in the evaluation system 2, the amount of the oxidized LDL can be rapidly evaluated.

The evaluation method according to this embodiment includes: acquiring information on a change amount of an absorbance per unit time, the change amount being obtained by a measurement method including causing a peroxidase to act on hydrogen peroxide, which is produced by solubilizing an LDL in a specimen with a surfactant that acts on the LDL, and subjecting the resultant to an oxidation reaction with a cholesterol oxidase, to introduce the hydrogen peroxide to a color reaction, followed by measurement of a change of the absorbance with time; and evaluating an amount of an oxidized LDL in the specimen based on the change amount.

Thus, in the evaluation method according to this embodiment, the amount of the oxidized LDL in the specimen can be evaluated based on the change amount of the absorbance per unit time measurable with the autoanalyzer 20. Accordingly, in the evaluation method according to this embodiment, the amount of the oxidized LDL can be rapidly evaluated.

### 6. Modification Examples

### 6.1. First Modification Example

In the embodiments described above, the evaluating unit 404 has evaluated the oxidized LDL amount through the determination of a value (reaction delay indicator), which is obtained by multiplying the change amount of the absorbance per unit time by a constant, or a value (oxidized LDL ratio), which is obtained by dividing the value by the LDL cholesterol concentration of the specimen, as an evaluation value.

In contrast, the evaluating unit 404 may evaluate the oxidized LDL amount through the judgment of whether or not such evaluation value exceeds a reference value. For example, the evaluating unit 404 may use the value of the oxidized LDL ratio at an incubation time of 0 days illustrated in FIG. 12 as the reference value to judge whether or not the oxidized LDL ratio of an actual specimen exceeds the reference value. The evaluating unit 404 may produce, for example, the list of specimens exceeding the reference value as evaluation results.

### 6.2. Second Modification Example

In the embodiments described above, the controlling unit 240 of the autoanalyzer 20 has functioned as the differential operation unit configured to calculate the change amount of the absorbance per unit time through the differential analysis of the change of the absorbance with time that is a result of measurement by the measuring unit. However, the acquiring unit 402 of the evaluation apparatus 40 may function as the differential operation unit.

For example, the autoanalyzer 20 outputs information on the change of the absorbance with time, and the acquiring unit 402 acquires the information on the change of the absorbance with time. The acquiring unit 402 performs the differential operation of the change of the absorbance with time to determine the change amount of the absorbance per unit time. Thus, the acquiring unit 402 can acquire the information on the change amount of the absorbance per unit time.

### 6.3. Third Modification Example

In the embodiments described above, as illustrated in FIG. 15, the evaluation system 2 has included the autoanalyzer 20, the evaluation apparatus 40, and the information processing terminal 60, and has been configured so that these components can be connected to each other through the communication network 4 such as the Internet, and an evaluation result has been displayed on the displaying unit of the information processing terminal 60.

In contrast, in the evaluation system 2, the autoanalyzer 20 and the evaluation apparatus 40 may be connected to each other through a local area network (LAN). In this case, the evaluation system 2 does not include the information processing terminal 60, and hence the evaluation result may be displayed on the displaying unit of the controlling unit 240 of the autoanalyzer 20 or the displaying unit 420 of the evaluation apparatus 40.

### 7. Other Embodiments

In the embodiments described above, the oxidized LDL amount has been evaluated as follows: the color reaction based on the reaction between the LDL and the reagent has been defined as the main reaction (first reaction) of the reagent, and the color reaction based on the reaction between the oxidized LDL and the reagent has been defined as the side reaction (second reaction) of the reagent; and the change amount of the absorbance of each of dyes produced by the main reaction and the side reaction per unit time has been measured, followed by the evaluation based on the change amount of the absorbance per unit time.

The evaluation method is applicable even to a specimen except a specimen containing an LDL and an oxidized LDL. That is, the evaluation method may include: causing a specimen containing a first substance and a second substance, and the reagent to react with each other, followed by the measurement of the change amount of a physical quantity of reaction products per unit time, the reaction products including a first reaction product produced by the first reaction between the first substance and the reagent, and a second reaction product produced by the second reaction between the second substance and the reagent; and evaluating the physical quantity of the second substance based on the change amount of the physical quantity of the reaction products per unit time.

Herein, in the embodiments described above, the first substance has been the LDL, and the first reaction product has been a dye (first dye) produced by the reaction between the LDL and the reagent. In addition, the second substance has been the oxidized LDL, and the second reaction product has been a dye (second dye) produced by the reaction between the oxidized LDL and the reagent. In contrast, the first substance and the second substance are not particularly limited as long as the first reaction product is produced by the first reaction between the first substance and the reagent, and the second reaction product is produced by the second reaction between the second substance and the reagent.

In addition, the first reaction and the second reaction are not limited to color reactions, and may each be any one of modification, oxidation, reduction, addition, bonding, degradation, condensation, and a solubilization reaction, or a combination thereof. Accordingly, the first reaction product and the second reaction product are not limited to dyes.

In addition, when each of the reaction products has a characteristic absorption wavelength, the physical quantity of the reaction product may be detected with an absorbance. Similarly, when each of the reaction products has a characteristic emission wavelength, Raman shift, chemical shift, or mass-to-charge ratio, the physical quantity of the reaction product may be detected by a method, such as fluorescence analysis, Raman spectroscopy, nuclear magnetic resonance spectrometry, or mass spectrometry, respectively.

For example, when the change of the physical quantity of the reaction products including the first reaction product and the second reaction product is represented by f(x), the following may be performed: the side reaction is represented as the derivative f'(x) thereof; and the physical quantity of the second substance is represented as the change amount of the physical quantity of the reaction products per unit time.

The evaluation value of the physical quantity of the second substance may be determined in the same manner as in the evaluation value of the oxidized LDL amount described above. That is, a value obtained by multiplying the change amount of the physical quantity of the reaction products per unit time by a constant (the constant is any number) or a value obtained by dividing the value by the amount (concentration) of the first substance may be used as the evaluation value of the physical quantity of the second substance.

The embodiments and the modification examples described above are merely examples, and the invention is not limited thereto. For example, the respective embodiments and the respective modification examples may be combined appropriately.

The invention is not limited to the above-described embodiments, and various modifications can be made. For example, the invention includes configurations that are substantially the same as the configurations described in the embodiments. Substantially the same configurations mean configurations having the same functions and methods, for example. The invention also includes configurations obtained by replacing non-essential elements of the configurations described in the embodiments with other elements. The invention further includes configurations obtained by adding known art to the configurations described in the embodiments.

## Claims

1. An evaluation apparatus, comprising:
an acquiring unit configured to acquire information on a change amount of an absorbance per unit time, the change amount being obtained by a measurement method including causing a peroxidase to act on hydrogen peroxide, which is produced by solubilizing an LDL in a specimen with a surfactant that acts on the LDL, and subjecting the resultant to an oxidation reaction with a cholesterol oxidase, to introduce the hydrogen peroxide to a color reaction, followed by measurement of a change of the absorbance with time; and
an evaluating unit configured to evaluate an amount of an oxidized LDL in the specimen based on the change amount.

2. The evaluation apparatus according to claim 1, wherein the evaluating unit is configured to determine an evaluation value of the amount of the oxidized LDL based on a value obtained by multiplying the change amount by a constant.

3. The evaluation apparatus according to claim 1, wherein the evaluating unit is configured to determine an evaluation value of the amount of the oxidized LDL based on a value obtained by dividing the change amount by an LDL cholesterol concentration of the specimen.

4. The evaluation apparatus according to claim 3, wherein the evaluating unit is configured to judge whether or not the evaluation value exceeds a reference value.

5. The evaluation apparatus according to claim 3 or 4, further comprising a data-generating unit configured to generate data for causing a displaying unit to display at least one of the LDL cholesterol concentration of the specimen, the change amount, or the evaluation value.

6. The evaluation apparatus according to any one of claims 1 to 5, wherein the change amount is a change amount of the absorbance per unit time during a period from a time point dating back a predetermined time from an endpoint of the reaction to the endpoint of the reaction.

7. A system, comprising:
a measuring unit configured to cause a peroxidase to act on hydrogen peroxide, which is produced by solubilizing an LDL in a specimen with a surfactant that acts on the LDL, and subjecting the resultant to an oxidation reaction with a cholesterol oxidase, to introduce the hydrogen peroxide to a color reaction, followed by measurement of a change of an absorbance with time to output data on the change of the absorbance with time;
a differential operation unit configured to determine a change amount of the absorbance per unit time from the data; and
an evaluating unit configured to evaluate an amount of an oxidized LDL in the specimen based on the change amount.

8. An evaluation method, comprising:
acquiring information on a change amount of an absorbance per unit time, the change amount being obtained by a measurement method including causing a peroxidase to act on hydrogen peroxide, which is produced by solubilizing an LDL in a specimen with a surfactant that acts on the LDL, and subjecting the resultant to an oxidation reaction with a cholesterol oxidase, to introduce the hydrogen peroxide to a color reaction, followed by measurement of a change of the absorbance with time; and
evaluating an amount of an oxidized LDL in the specimen based on the change amount.

9. An evaluation method, comprising:
causing a specimen containing a first substance and a second substance, and a reagent to react with each other, followed by measurement of a change amount of a physical quantity of reaction products per unit time, the reaction products including a first reaction product produced by a first reaction between the first substance and the reagent, and a second reaction product produced by a second reaction between the second substance and the reagent; and
evaluating a physical quantity of the second substance based on the change amount.

10. The evaluation method according to claim 9,
wherein the specimen is a body fluid,
wherein the first substance is an LDL,
wherein the second substance is an oxidized LDL,
wherein the first reaction is a color reaction based on a reaction between the LDL and the reagent, and
wherein the second reaction is a color reaction based on a reaction between the oxidized LDL and the reagent.

11. The evaluation method according to claim 10,
wherein the first reaction product is a first dye produced by the reaction between the LDL and the reagent, and
wherein the second reaction product is a second dye produced by the reaction between the oxidized LDL and the reagent.
